# EUROPÄISCHE PATENTSCHRIFT

(11) **EP 2 737 568 B1**
(45) Veröffentlichungstag und Bekanntmachung des Hinweises auf die Patenterteilung: **17.06.2015**
(21) Anmeldenummer: 12738464.2
(22) Anmeldetag: 25.07.2012
(51) Int. Cl.: C07C 309/10, H01G 11/06, H01G 11/62, H01M 10/052, H01M 10/0568, H01M 10/0569

(54) **LITHIUM-2-METHOXY-1,1,2,2-TETRAFLUOR-ETHANSULFONAT UND DESSEN VERWENDUNG ALS LEITSALZ IN LITHIUM-BASIERTEN ENERGIESPEICHERN**
LITHIUM-2-METHOXY-1,1,2,2-TETRAFLUORO-ETHANESULFONATE AND USE THEREOF AS CONDUCTIVE SALT IN LITHIUM-BASED ENERGY ACCUMULATORS
LITHIUM-2-MÉTHOXY-1,1,2,2-TÉTRAFLUORO-ÉTHANESULFONATE ET SON UTILISATION COMME SEL CONDUCTEUR DANS DES ACCUMULATEURS D'ÉNERGIE À BASE DE LITHIUM

(30) Priorität: 26.07.2011 DE 102011052156
(43) Veröffentlichungstag der Anmeldung: 04.06.2014
(73) Patentinhaber: Westfälische Wilhelms-Universität Münster, 48149 Münster (DE); Jacobs University Bremen gGmbH, 28759 Bremen (DE)
(72) Erfinder: RÖSCHENTHALER, Gerd-Volker, 28357 Bremen (DE); WINTER, Martin, 48149 Münster (DE); VLASOV, Katja, 68199 Mannheim (DE); KALINOVICH, Nataliya, 28755 Bremen (DE); SCHREINER, Christian, 86485 Biberbach (DE); SCHMITZ, Raphael Wilhelm, 48151 Münster (DE); MÜLLER, Romek Ansgar, 85057 Ingolstadt (DE); SCHMITZ, René, 68163 Mannheim (DE); LEX-BALDUCCI, Alexandra, 48159 Münster (DE); KUNZE, Miriam, 37444 St. Andreasberg (DE)
(74) Vertreter: Michalski Hüttermann & Partner Patentanwälte mbB
(86) Internationale Anmeldenummer: PCT/EP2012/064584
(87) Internationale Veröffentlichungsnummer: WO 2013/014180

(56) Entgegenhaltungen:
- EP-A1- 1 598 896
- EP-A2- 1 220 344
- WO-A1-01/78183
- WO-A1-2012/084066
- JP-A- 10 092 222
- PAILLARD ET AL: "Electrochemical investigation of polymer electrolytes based on lithium 2-(phenylsulfanyl)-1,1,2,2-tetrafluoro-eth ansulfonate", ELECTROCHIMICA ACTA, ELSEVIER SCIENCE PUBLISHERS, BARKING, GB, Bd. 53, Nr. 4, 30. Oktober 2007 (2007-10-30), Seiten 1439-1443, XP022321985, ISSN: 0013-4686, DOI: 10.1016/J.ELECTACTA.2007.05.027
- ARVAI R ET AL: "New aryl-containing fluorinated sulfonic acids and their ammonium salts, useful as electrolytes for fuel cells or ionic liquids", JOURNAL OF FLUORINE CHEMISTRY, ELSEVIER, NL, Bd. 129, Nr. 10, 1. Oktober 2008 (2008-10-01), Seiten 1029-1035, XP025468252, ISSN: 0022-1139, DOI: 10.1016/J.JFLUCHEM.2008.06.009 [gefunden am 2008-06-20]

## Beschreibung

Die Erfindung betrifft Lithium-2-methoxy-1,1,2,2-tetrafluor-ethansulfonat sowie die Verwendung von Lithium-2-methoxy-1,1,2,2-tetrafluor-ethansulfonat als Leitsalz in Lithium-basierten Energiespeichern. Weiter betrifft die Erfindung ionische Flüssigkeiten umfassend 2-Methoxy-1,1,2,2-tetrafluor-ethansulfonat als Anion.

Die Lithium-Ionen-Technologie ist die führende Technologie auf dem Gebiet der wiederaufladbaren Batteriespeichersysteme für die portable Elektronik. Lithium-Ionen Batterien werden als Speichersysteme in Mobiltelefonen, Cam-Cordern, Laptops und seit einiger Zeit auch in Akkuwerkzeugen eingesetzt. Der nächste angestrebte Schritt ist der Einsatz von Lithium-Ionen Batterien in größeren Systemen, wie in Automobilen oder als stationäre Energiespeicher für erneuerbare Energien. Aufgrund ihrer hohen Zellspannung, ihrer überlegenen Energie- und Leistungsdichte sowie ihrer ausgesprochen niedrigen Selbstentladung haben Lithium-Ionen Batterien ein hohes Potential für diese Anwendungen. Jedoch erfüllen die kommerziell erhältlichen Batterien noch nicht die erhöhten Sicherheitsanforderungen für große Systeme. Eine wichtige Rolle spielen hierbei die thermische und chemische Stabilität der verwendeten Flüssigelektrolyte. Da beim Entladen von Lithium-Ionen Batterien Wärme entsteht, ist vor allem für große Systeme mit einigen hundert bis tausend Kilowattstunden an gespeicherter Energie eine ausreichende thermische Stabilität der verwendeten Elektrolyte eine unbedingt notwendige Voraussetzung für ihre Anwendung.

Derzeit wird in kommerziell erhältlichen Batterien als Leitsalz Lithiumhexafluorphosphat (LiPF₆) verwendet. Lithiumhexafluorphosphat weist eine relativ hohe Leitfähigkeit auf und ist in der Lage eine Passivierungsschicht, die sogenannte Solid Electrolyte Interphase (SEI) auf Graphitelektroden auszubilden. Lithiumhexafluorphosphat weist jedoch erhebliche Nachteile aufgrund seiner geringen thermischen und chemischen Stabilität auf. So ist bekannt, dass sich LiPF₆ mit Spuren von Wasser und anderen protischen Verbindungen wie Alkoholen, die in Lithium-Batterien nicht gänzlich vermeidbar sind und beispielsweise im ppm-Bereich in Lösungsmitteln vorkommen, reagiert und die toxischen Verbindungen POF₃ und HF, welche die Auflösung der als Kathodenmaterialien verwendeten Spinelle LiₓMn₂O₄ sowie die Degradation der Passivierungsschichten sowohl auf der Anode wie auch auf der Kathode beschleunigen, bildet. Diese Reaktion wird durch mäßig erhöhte Temperaturen beschleunigt. Dadurch kommt es zu einem schnellen Verlust der Kapazität der Zelle, was sich in einer verkürzten Lebensdauer der Zelle auswirkt.

Daher gibt es intensive Bemühungen, alternative Lithiumsalze zu entwickeln, die LiPF₆ als Leitsalz ersetzen können. Die in den letzten Jahren entwickelten Lithiumsalze sind häufig komplexe Bor- oder Phosphorhaltige Anionen mit nicht-aromatischen Chelatbildnern wie Oxalat, beispielsweise das in der DE 198 29 030 C1 offenbarte Lithium-Bis(oxalato)borat (LiBOB). Nachteilig ist jedoch, dass Bis(oxalato)borat eine nur geringe Löslichkeit in den üblichen als Lösungsmittel in Elektrolyten verwendeten Carbonaten aufweist. Weiter weisen LiBOB-basierte Elektrolyte im Vergleich zu LiPF₆ eine geringere Leitfähigkeit und eine höhere Viskosität auf. Insbesondere weisen Bis(oxalato)borat-Elektrolyte nur eine geringe Leitfähigkeit bei niedrigen Temperaturen auf. Darüber hinaus ist die Herstellung von Bis(oxalato)borat ausreichender Reinheit aufwendig, da Verunreinigungen mit Oxalat und Carboxylat bei erhöhten Temperaturen zum Austreten von Gasen aus den Zellen führt. Ein weiterer Nachteil bei der Verwendung von Lithium-bis(oxalato)borat ist, dass eine zu starke SEI ausgebildet wird, wodurch der Widerstand der Zelle erhöht wird.

Trotz einer Vielzahl an Salzen und Lösungsmitteln wurde noch kein geeigneter Ersatz für LiPF₆ als Leitsalz in Carbonatgemischen gefunden. Daher besteht ein Bedarf an alternativen Lithiumsalzen.

Der vorliegenden Erfindung lag daher die Aufgabe zu Grunde, eine Verbindung zur Verfügung zu stellen, die mindestens einen der vorgenannten Nachteile des Standes der Technik überwindet. Insbesondere lag der vorliegenden Erfindung die Aufgabe zu Grunde eine in Carbonatgemischen als Leitsalz geeignete Lithiumverbindung zur Verfügung zu stellen.

Diese Aufgabe wird gelöst durch die Verbindung Lithium-2-methoxy-1,1,2,2-tetrafluor-ethansulfonat. Lithium-2-methoxy-1,1,2,2-tetrafluor-ethansulfonat wird auch als Lithium-1,1,2,2-tetrafluor-2-methoxyethansulfonat bezeichnet.

Überraschend wurde gefunden, dass Elektrolyte, welche die erfindungsgemäße Verbindung Lithium-2-methoxy-1,1,2,2-tetrafluor-ethansulfonat als Lithiumsalz enthalten, selbst bei Temperaturen von 95°C keine HF-Entwicklung zeigen. So wurde in thermischen Alterungsversuchen festgestellt, dass für diese Elektrolyte selbst nach einer Lagerung bei 95°C für zwei Wochen, im Gegensatz zu LiPF₆-basierten Elektrolyten, in Kernspinresonanz (NMR)-Spektren keine HF-Entwicklung sichtbar war. Weiterhin konnte festgestellt werden, dass bei Elektrolyte enthaltend Lithium-2-methoxy-1,1,2,2-tetrafluor-ethansulfonat nach einer Lagerung bei 95°C für zwei Wochen, im Gegensatz zu LiPF₆ enthaltenden Elektrolyten, keine Carbonatzersetzungsprodukte im Elektrolyten feststellbar waren. Eine gute thermische Stabilität stellt ist einen großen Vorteil von Lithium-2-methoxy-1,1,2,2-tetrafluor-ethansulfonat dar. Dies ermöglicht einen erweiterten Temperaturbereich für die Verwendung von Lithium-2-methoxy-1,1,2,2-tetrafluor-ethansulfonat als Lithiumleitsalz. Ebenso zeigen Zellen unter Verwendung von Lithium-2-methoxy-1,1,2,2-tetrafluor-ethansulfonat als Leitsalz eine hervorragende Zyklenstabilität.

Weiterhin zeigt Lithium-2-methoxy-1,1,2,2-tetrafluor-ethansulfonat ebenfalls eine gute elektrochemische Stabilität. So zeigt Lithium-2-methoxy-1,1,2,2-tetrafluor-ethansulfonat in einem Gemisch aus Ethylencarbonat (EC) und Diethylcarbonat (DEC) eine anodische Stabilität von 5,6 V. Dieser Wert liegt in gleicher Höhe wie die mit LiPF₆ in Carbonatgemischen erzielbaren 5,9 V und ist ausreichend für die Verwendung mit Hochvoltkathodenmaterialien. Insbesondere zeigen Korrosionsmessungen, dass Lithium-2-methoxy-1,1,2,2-tetrafluor-ethansulfonat keine Korrosion des an der Kathodenseite als Stromsammler eingesetzten Aluminiums zeigt, sondern wie LiPF₆ eine Schutzschicht auf Aluminium ausbildet.

Lithium-2-methoxy-1,1,2,2-tetrafluor-ethansulfonat weist neben der positiven Eigenschaft, keine HF-Entwicklung zu zeigen, eine gute Lithium-Ionen Leitfähigkeit, eine hohe elektrochemische Stabilität und gute SEI-Filmbildungseigenschaften auf. Außerdem ist Lithium-2-methoxy-1,1,2,2-tetrafluor-ethansulfonat nicht brennbar und in einem weiten Temperaturbereich anwendbar.

Durch eine Vermeidung einer HF-Entwicklung auch in Gegenwart von größeren Mengen an Wasser durch Lithium-2-methoxy-1,1,2,2-tetrafluor-ethansulfonat, kann im Gegensatz zu LiPF₆-Elektrolyten eine erhebliche Verbesserung der Betriebssicherheit zur Verfügung gestellt werden.

Lithium-2-methoxy-1,1,2,2-tetrafluor-ethansulfonat zeigt insbesondere den Vorteil, auch bei erhöhten Temperaturen kein HF zu entwickeln, und eine geringere Toxizität der Verbrennungsprodukte als LiPF₆. In vorteilhafter Weise ermöglicht dies einen breiteren Temperaturbereich der Verwendung beispielsweise bei Lithium-Ionen Akkumulatoren bei vergleichbarer Leistung. Somit ist Lithium-2-methoxy-1,1,2,2-tetrafluor-ethansulfonat in vorteilhafter Weise als Leitsalz für kommerzielle Lithium-Ionen Batterien verwendbar.

Ein weiterer Gegenstand der vorliegenden Erfindung betrifft die Verwendung von Lithium-2-methoxy-1,1,2,2-tetrafluor-ethansulfonat in Lithium-basierten Energiespeichern, insbesondere als Leitsalz. Primäre und sekundäre Lithium-basierte Energiespeicher sind vorzugsweise ausgewählt aus der Gruppe umfassend Lithium-Batterien, Lithium-Ionen-Batterien, Lithium-Ionen-Akkumulatoren, Lithium-Polymer-Batterien und/oder Lithium-Ionen-Kondensatoren. Insbesondere eignet sich Lithium-2-methoxy-1,1,2,2-tetrafluor-ethansulfonat als Leitsalz für eine Lithium-Ionen Batterie oder einen Lithium-Ionen Akkumulator. Weiter eignet sich Lithium-2-methoxy-1,1,2,2-tetrafluor-ethansulfonat als Leitsalz für Lithium-basierte Energiespeicher, die als Weiterentwicklungen der Lithium-Ionen-Akkumulatoren bezeichnet werden, vorzugsweise ausgewählt aus der Gruppe umfassend Lithium-Titanat-Akkumulatoren, Lithium-Luft-Akkumulatoren, Lithium-Mangan-Akkumulatoren, Lithium-Eisen-Phosphat-Akkumulatoren, Lithium-Eisen-Mangan-Phosphat-Akkumulatoren, Lithium-Eisen-Yttrium-Phosphat-Akkumulatoren, Lithium-Schwefel-Akkumulatoren, Lithium-Nickel-Cobalt-Mangan-Oxid-Akkumulatoren, Lithium-Nickel-Cobalt-Aluminium-Oxid-Akkumulatoren und Zinn-Schwefel-Lithium-Akkumulatoren.

Der Ladungstransport in Lithium-basierten elektrochemischen Energiespeichern erfolgt über einen Elektrolyten. Ein flüssiger Elektrolyt wird üblicherweise im Wesentlichen aus einem in einem Lösungsmittel gelösten Lithiumleitsalz ausgebildet.

Ein weiterer Gegenstand der Erfindung betrifft einen Elektrolyt für einen Lithium-basierten Energiespeicher umfassend Lithium-2-methoxy-1,1,2,2-tetrafluor-ethansulfonat. Lithium-basierte Energiespeicher umfassend Lithium-2-methoxy-1,1,2,2-tetrafluor-ethansulfonat als Leitsalz weisen insbesondere den Vorteil einer guten thermischen und elektrochemischen Stabilität auf.

Vorzugsweise umfasst der Elektrolyt ein aprotisches Lösungsmittel, eine ionische Flüssigkeit und/oder eine Polymermatrix. Vorzugsweise umfasst ein Elektrolyt ein aprotisches Lösungsmittel und Lithium-2-methoxy-1,1,2,2-tetrafluor-ethansulfonat. Es konnte festgestellt werden, dass Lithium-2-methoxy-1,1,2,2-tetrafluor-ethansulfonat in aprotischen Lösungsmitteln insbesondere cyclischen oder linearen Carbonaten eine gute Löslichkeit aufweist. Dies ermöglicht in vorteilhafter Weise die Verwendung von Lithium-2-methoxy-1,1,2,2-tetrafluor-ethansulfonat in den in kommerziellen Lithium-Ionen Batterien eingesetzten Flüssigelektrolyten.

In bevorzugten Ausführungsformen ist das aprotische Lösungsmittel ausgewählt aus der Gruppe umfassend Ethylencarbonat, Propylencarbonat, Diethylcarbonat, Dimethylcarbonat, Ethylmethylcarbonat, Acetonitril, Glutaronitril, Adiponitril, Pimelonitril, gamma-Butyrolacton, gamma-Valerolacton, Dimethoxyethan, 1,3-Dioxalan, Methylacetat und/oder Mischung davon. Bevorzugt sind cyclische Carbonate wie Ethylencarbonat oder Propylencarbonat, und/oder lineare Carbonate wie Diethylcarbonat, Dimethylcarbonat oder Ethylmethylcarbonat. Vorzugsweise ist das aprotische Lösungsmittel ausgewählt aus der Gruppe umfassend Ethylencarbonat, Diethylcarbonat, Dimethylcarbonat und/oder deren Mischungen.

Bevorzugt sind Mischungen von Ethylencarbonat und wenigstens einem weiteren aprotischen Lösungsmittel, besonders bevorzugt mit Diethylcarbonat oder Dimethylcarbonat. In bevorzugten Ausführungsformen ist das aprotische Lösungsmittel eine Mischung aus Ethylencarbonat und wenigstens einen weiteren aprotischen Lösungsmittel vorzugsweise Diethylcarbonat. Bevorzugt liegt das Verhältnis von Ethylencarbonat und dem wenigstens einen weiteren aprotischen Lösungsmittel vorzugsweise Diethylcarbonat im Bereich von ≥ 1:9 bis ≤ 9:1, bevorzugt im Bereich von ≥ 3:7 bis ≤ 7:3, vorzugsweise im Bereich von ≥ 3:7 bis ≤ 1:1. Wenn nicht abweichend angegeben bezieht sich das Verhältnis auf die Gewichtsanteile der Lösungsmittel.

In einem Lösungsmittelgemisch Ethylencarbonat und Diethylcarbonat (EC:DEC) im Verhältnis 1:1 konnte in vorteilhafter Weise die höchste Leitfähigkeit in einem Temperaturbereich von -20°C - +60°C erreicht werden.

Als vielversprechende Elektrolytmaterialien haben sich weiterhin ionische Flüssigkeiten erwiesen, da diese eine hohe thermische wie elektrochemische Stabilität mit einer hohen ionischen Leitfähigkeit vereinen. Insbesondere ist dies vorteilhaft zur Verwendung mit Lithium-2-methoxy-1,1,2, 2-tetrafluor-ethansulfonat.

Bevorzugte ionische Flüssigkeiten umfassen ein Kation ausgewählt aus der Gruppe umfassend 1-Ethyl-3-methylimidazolium (EMI⁺), 1,2-Dimethyl-3-propylimidazolium (DMPI⁺), 1,2-Diethyl-3,5-dimethylimidazolium (DEDMI⁺), Trimethyl-*n*-hexylammonium (TMHA⁺), *N*-alkyl-*N*-methylpyrrolidinium (PYR_{1R}⁺), *N*-alkyl-*N*-methylpiperidinium (PIP_{1R}⁺) und/oder *N*-alkyl-*N*-methylmorpholinium (MORP_{1R}⁺) und ein Anion ausgewählt aus der Gruppe umfassend Bis(trifluormethansulfonyl)imid (TFSI⁻), Bis(pentafluorethansulfonyl)imid (BETI⁻), Bis(fluorsulfonyl)imid (FSI⁻), 2,2,2-Trifluor-*N*-(trifluormethansulfonyl)acetamid (TSAC⁻), Tetrafluorborat (BF₄⁻), Pentafluorethanetrifluoroborate (C₂F₅BF₃⁻), Hexafluorphosphat (PF₆⁻) und/oder Tri(pentafluorethan)trifluorphosphat ((C₂F₅)₃PF₃⁻). Bevorzugte *N*-alkyl-*N*-methylpyrrolidinium (PYR_{1R}⁺) Kationen sind ausgewählt aus der Gruppe umfassend N-butyl-N-methylpyrrolidinium (PYR₁₄⁺) und/oder N-methyl-N-propylpyrrolidinium (PYR₁₃⁺). Bevorzugte ionische Flüssigkeiten sind ausgewählt aus der Gruppe umfassend *N*-butyl-*N*-methylpyrrolidiniumbis(trifluoromethansulfonyl)imid (PYR₁₄TFSI) und/oder *N*-methyl-*N*-propylpyrrolidiniumbis(trifluoromethansulfonyl)imid (PYR₁₃TFSI).

Weitere geeignete Elektrolytmaterialien sind Polymerelektrolyte, wobei der Polymerelektrolyt als Gelpolymerelektrolyt oder fester Polymerelektrolyt vorliegen kann.

Feste Polyelektrolyte zeigen gute Eigenschaften bezüglich der Anforderungen an zukünftige Akkumulatorgenerationen. Sie ermöglichen einen solvensfreien Aufbau, der einfach herzustellen und vielfältig in der Form ist. Darüber hinaus kann die Energiedichte gesteigert werden, denn es entfällt der Dreischichtaufbau aus Elektrolyt-Separator-Elektrolyt, sodass lediglich eine dünne Polymerfolie zwischen den Elektroden benötigt wird. Festelektrolyte sind in der Regel chemisch und elektrochemisch stabil gegenüber Elektrodenmaterialien und treten weiterhin nicht aus der Zelle aus. Gelpolymerelektrolyte umfassen meist ein aprotisches Lösungsmittel und eine Polymermatrix.

Bevorzugte Polymere für feste Polymerelektrolyte und Gelpolymerelektrolyte sind ausgewählt aus der Gruppe umfassend Homo- oder Copolymere von Polyethylenoxid (PEO), Polypropylenoxid (PPO), Polyvinylidenfluorid (PVdF), Polyvinylidenfluoridhexafluorpropylen (PVdF-HFP), Polyacrylnitril (PAN), Polymethylmethacrylat (PMMA), Polyethylmethacrylat (PEMA), Polyvinylacetat (PVAc), Polyvinylchlorid (PVC), Polyphophazene, Polysiloxane, Polyvinylalkohol (PVA) und/oder Homo- und (Block-) Copolymere umfassend funktionelle Seitenketten ausgewählt aus der Gruppe umfassend Ethylenoxid, Propylenoxid, Acrylnitril und/oder Siloxane.

Vorzugsweise liegt Lithium-2-methoxy-1,1,2,2-tetrafluor-ethansulfonat in dem Lösungsmittel gelöst vor. In bevorzugten Ausführungsformen liegt die Konzentration an Lithium-2-methoxy-1,1,2,2-tetrafluor-ethansulfonat im Elektrolyten im Bereich von ≥ 0,5 M bis ≤ 2,5 M, vorzugsweise im Bereich von ≥ 0,65 M bis ≤ 2 M, besonders bevorzugt im Bereich von ≥ 1 M bis ≤ 1,5 M. In vorteilhafter Weise führt eine solche Konzentration an Lithium-2-methoxy-1,1,2,2-tetrafluor-ethansulfonat im Elektrolyten zu einer guten Leitfähigkeit.

Ein weiterer Gegenstand der Erfindung betrifft einen Lithium-basierten Energiespeicher, vorzugsweise eine Lithium-Batterie, Lithium-Ionen-Batterie, Lithium-Ionen-Akkumulator, Lithium-Polymer-Batterie oder Lithium-Ionen-Kondensator umfassend Lithium-2-methoxy-1,1,2,2-tetrafluor-ethansulfonat. Ebenfalls bevorzugt ist ein Lithium-basierten Energiespeicher, vorzugsweise eine Lithium-Batterie, Lithium-Ionen-Batterie, Lithium-Ionen-Akkumulator, Lithium-Polymer-Batterie oder Lithium-Ionen-Kondensator, umfassend einen erfindungsgemäßen Elektrolyten umfassend Lithium-2-methoxy-1,1,2,2-tetrafluor-ethansulfonat.

Die Lithium-basierten Energiespeicher eignen sich für alle Anwendungsgebiete, insbesondere auch für größere Systeme wie Automobile oder als stationäre Energiespeicher für erneuerbare Energien.

Lithium-2-methoxy-1,1,2,2-tetrafluor-ethansulfonat ist nach üblichen Synthesemethoden herstellbar. Bevorzugt ist ein Verfahren zur Herstellung von Lithium-2-methoxy-1,1,2,2-tetrafluor-ethansulfonat umfassend die folgenden Schritte:
a) Umsetzen von Difluorfluorsulfonylacetylfluorid mit Triethylamintrihydrofluorid oder Tetramethylammoniumfluorid zu Triethylammonium-2-sulfonylfluorid-tetrafluorethanolat oder Tetramethylammonium-2-sulfonylfluorid-tetrafluorethanolat,
b) Methylierung von Triethylammonium-2-sulfonylfluorid-tetrafluorethanolat oder Tetramethylammonium-2-sulfonylfluorid-tetrafluorethanolat zu 2-Methoxy-1,1,2,2-tetrafluor-ethansulfonylfluorid, und
c) Umsetzen von 2-Methoxy-1,1,2,2-tetrafluor-ethansulfonylfluorid mit Lithiumhydroxid zu Lithium-2-methoxy-1,1,2,2-tetrafluorethansulfonat.

Die Synthese von Lithium-2-methoxy-1,1,2,2-tetrafluor-ethansulfonat erfolgt vorzugsweise ausgehend von Difluor-fluorsulfonyl-acetylfluorid. Dieses wird mit Triethylamintrihydrofluorid oder Tetramethylammoniumfluorid umgesetzt. Bevorzugt wird die Reaktion in Dichlormethan oder Acetonitril durchgeführt. Die Umsetzung von Difluor-fluorsulfonyl-acetylfluorid mit Triethylamintrihydrofluorid wird vorzugsweise in einer Mischung von Triethylamintrihydrofluorid und Triethylamin durchgeführt. Bevorzugt ist ein 1:2-Gemisch von Triethylamintrihydrofluorid und Triethylamin. Das Lösungsmittel ist vorzugsweise Dichlormethan. Die Umsetzung von Difluor-fluorsulfonyl-acetylfluorid mit Tetramethylammoniumfluorid wird vorzugsweise in Acetonitril durchgeführt. Vorzugsweise werden Triethylamintrihydrofluorid oder Tetramethylammoniumfluorid im Lösungsmittel gelöst, die Mischung beispielsweise mit flüssigem Stickstoff gekühlt, und Difluor-fluorsulfonyl-acetylfluorid einkondensiert. Bevorzugte Reaktionstemperaturen für die Umsetzung zu Triethylammonium-2-sulfonylfluorid-tetrafluorethanolat oder Tetramethylammonium-2-sulfonylfluorid-tetrafluorethanolat liegen bei 20°C.

Die Methylierung von Triethylammonium-2-sulfonylfluorid-tetrafluorethanolat oder Tetramethylammonium-2-sulfonylfluorid-tetrafluorethanolat zu 2-Methoxy-1,1,2,2-tetrafluor-ethansulfonylfluorid in Schritt b) wird vorzugsweise mittels Methylierungsmitteln ausgewählt aus der Gruppe umfassend Dimethylsulfat und/oder Trifluormethansulfonsäuremethylester (Methyltriflat) durchgeführt. Insbesondere Methyltriflat ist ein starkes Methylierungsreagenz. Vorzugsweise wird Triethylammonium-2-sulfonylfluorid-tetrafluorethanolat unter Verwendung von Dimethylsulfat methyliert. Vorzugsweise wird Tetramethylammonium-2-sulfonylfluorid-tetrafluorethanolat unter Verwendung von Trifluormethansulfonsäuremethylester methyliert.

Das Umsetzen von 2-Methoxy-1,1,2,2-tetrafluor-ethansulfonylfluorid mit Lithiumhydroxid zu Lithium-2-methoxy-1,1,2,2-tetrafluorethansulfonat erfolgt vorzugsweise in Methanol. Beispielsweise wird Lithiumhydroxid unter Kühlung beispielsweise auf 0°C zugegeben und die Reaktionsmischung bei einer Temperatur im Bereich von 18°C bis 23°C reagiert. Lithium-2-methoxy-1,1,2,2-tetrafluorethansulfonat ist als Leitsalz in einem Elektrolyten umfassend beispielsweise eine ionische Flüssigkeit in Lithium-basierten Energiespeichern verwendbar.

Ionische Flüssigkeiten sind als nicht-wässrige Elektrolyten insbesondere für elektrochemische Verwendungen beispielsweise in Batterien oder elektrochemischen Kondensatoren verwendbar, jedoch auch für die Elektroplattierung, Katalyse oder chemische Reaktionen. Ionische Flüssigkeiten mit vorzugsweise breitem elektrochemischem Fenster und geringer Hygroskopie sind entsprechend nicht nur für elektrochemische Anwendungen vorteilhaft verwendbar.

Ein weiterer Gegenstand der Erfindung betrifft eine ionische Flüssigkeit umfassend 2-Methoxy-1,1,2,2-tetrafluor-ethansulfonat als Anion und ein organisches Kation ausgewählt aus der Gruppe umfassend Alkylammonium-, Pyridinium-, Pyrazolium-, Pyrrolium-, Pyrrolinium-, Piperidinium-, Pyrrolidinium-, Imidazolium- und/oder Sulfoniumverbindungen.

Vorzugsweise ist das Kation ausgewählt aus der Gruppe umfassend N-butyl-N-methylpyrrolidinium (PYR14), N-methyl-N-propylpyrrolidinium (PYR13), 1-Ethyl-3-methylimidazolium (EMIM), 1-Ethyl-2,3-dimethylimidazolium (EDiMIM) und/oder 1-Butyl-3-methylimidazolium (BMIM).

Ionische Flüssigkeit umfassend 2-Methoxy-1,1,2,2-tetrafluor-ethansulfonat als Anion und ein organisches Kation können in vorteilhafter Weise für elektrochemische Anwendungen Verwendung finden, beispielsweise in Kombination mit einem Lithiumsalz in Lithium-basierten Energiespeichern. Weiterhin sind Verwendungen in Solarzellen oder Brennstoffzellen vorteilhaft. In vorteilhafter Weise sind ionische Flüssigkeiten umfassend fluorierte Anionen auch als Hydraulikflüssigkeit oder inerte flüssige Verdünnungsmittel für hochreaktive Chemikalien verwendbar.

Beispiele und Figuren, die der Veranschaulichung der vorliegenden Erfindung dienen, sind nachstehend angegeben.

Hierbei zeigen die Figuren:
- Figur 1: zeigt die thermische Stabilität einer 1 M Lithium-2-methoxy-1,1,2,2-tetrafluor-ethansulfonat (LiB-7) Elektrolytlösung in EC:DEC 1:1 im Vergleich zu einer 1 M Lösung von LiPF₆ in EC:DEC 1:1 nach zweiwöchiger Lagerung bei 95°C, bestimmt mittels GC-MS.
- Figur 2: zeigt die temperaturabhängige Leitfähigkeit von Lithium-2-methoxy-1,1,2,2-tetrafluor-ethansulfonat (LiB-7) in verschiedenen Lösungsmittelgemischen.
- Figur 3: zeigt das elektrochemische Stabilitätsfenster von Lithium-2-methoxy-1,1,2,2-tetrafluor-ethansulfonat (LiB-7) in EC:DEC 1:1 und LiPF₆ in EC:DEC 3:7.
- Figur 4: zeigt Korrosionsmessungen auf Aluminium-Folie mit Lithium-2-methoxy-1,1,2,2-tetrafluor-ethansulfonat (LiB-7), LiPF₆ und Lithium Bis(Trifluoromethansulfonyl)-imid in EC:DEC 3:7.
- Figur 5: zeigt eine Konstantstromzyklisierung von Graphit-NCA Swagelok^{®}-Zellen geladen mit einer C-Rate von 1C und einer Stunde Konstantspannung und entladen mit einer C-Rate von 1C (Figur 5A); C-Raten-Test (Figur 5B); Konstantstromzyklisierung nach C-Raten-Test (Figur 5C).

### Beispiel 1

### Herstellung von Lithium-2-methoxy-1,1,2,2-tetrafluor-ethansulfonat

### a) Herstellung von Triethylammonium-2-sulfonylfluorid-tetrafluorethanolat

21 mmol (2,12 g) Triethylamin (ACROS, 99%) und 11 mmol Triethylamintrihydrofluorid (ALDRICH, 98%) wurden in 20 ml trockenem Dichlormethan (ROTH) gelöst. Die Lösung wurde mit flüssigem Stickstoff auf -196°C gebracht und 32 mmol Difluor-fluorsulfonyl-acetylfluorid (Synquest, 99,8%) wurden einkondensiert. Das Reaktionsgemisch wurde 3 Stunden bei 20°C gerührt. Flüchtige Bestandteile wurden im Vakuum abgezogen. Triethylammonium-2-sulfonylfluorid-tetrafluorethanolat wurde als gelbliches Öl erhalten.

### b) Herstellung von 2-Methoxy 1,1,2,2-tetrafluor-ethansulfonylfluorid

32 mmol Triethylammonium-2-sulfonylfluorid-tetrafluorethanolat aus Schritt a) wurden in 20 ml trockenem 2,2'-Dimethoxydiethylether (Diglyme, Aldrich) gelöst. Bei 0°C wurden 39 mmol Dimethylsulfat (ACROS) langsam zugegeben. Das Reaktionsgemisch wurde 7 Stunden auf 50°C erhitzt. Anschließen wurde das Produkt unter Normaldruck fraktionierend destilliert. 2-Methoxy-1,1,2,2-tetrafluor-ethansulfonylfluorid wurde als farblose Flüssigkeit erhalten.

### c) Herstellung von Lithium-2-methoxy-1,1,2,2-tetrafluor-ethansulfonat

21 mmol (3,78 g) 2-Methoxy-1,1,2,2-tetrafluor-ethanesulfonylfluorid aus Schritt b) wurden in Methanol (ROTH) gelöst. Bei 0°C wurde ein 2n Äquivalent Lithiumhydroxid (ACROS, 98%) zugegeben. Die Suspension wurde 2 Stunden bei Raumtemperatur gerührt. Die Suspension wurde 15 Minuten zentrifugiert, die flüssige Phase abdekantiert und das Lösungsmittel abgezogen. Das Produkt wurde im Vakuum bei 60°C 6 Stunden getrocknet. Lithium-2-methoxy-1,1,2,2-tetrafluor-ethansulfonat wurde in Form weißer Kristalle erhalten.

### Beispiel 2

### Herstellung von Lithium-2-methoxy-1,1,2,2-tetrafluor-ethansulfonat

### a) Herstellung von Tetramethylammonium-2-sulfonylfluorid-tetrafluorethanolat

10 mmol Tetramethylammoniumfluorid (99,9%) wurden in 20 ml trockenem Acetonitril (ROTH) gelöst. Die Lösung wurde mit flüssigem Stickstoff auf -196°C gebracht und 10 mmol Difluor-fluorsulfonyl-acetylfluorid einkondensiert. Das Reaktionsgemisch wurde 5 Stunden bei 20°C gerührt. Die flüchtigen Bestandteile wurden anschließend im Vakuum abgezogen.

Der zurückgebliebene Feststoff wurde mit 2 Mal 5 ml Diethylether gewaschen und im Vakuum getrocknet. Tetramethylammonium-2-sulfonylfluorid-tetrafluorethanolat wurde in Form farbloser Kristalle erhalten.

### b) Herstellung von 2-Methoxy 1,1,2,2-tetrafluor-ethansulfonylfluorid

20 mmol Tetramethylammonium-2-sulfonylfluorid-tetrafluorethanolat aus Schritt a) wurden in 20 ml trockenem Acetonitril gelöst. Bei 0°C wurden 22 mmol Trifluormethansulfonsäure-methylester (Methyltriflat) (ABCR, 98%) langsam zugegeben. Das Reaktionsgemisch wurde 5 Stunden bei Raumtemperatur gerührt. Anschließen wurde das Produkt unter Normaldruck fraktionierend destilliert. 2-Methoxy 1,1,2,2-tetrafluor-ethansulfonylfluorid wurde als farblose Flüssigkeit erhalten.

### c) Herstellung von Lithium-2-methoxy-1,1,2,2-tetrafluor-ethansulfonat

21 mmol (3,78 g) 2-Methoxy-1,1,2,2-tetrafluor-ethansulfonylfluorid aus Schritt b) wurden in Methanol gelöst. Bei 0°C wurde ein 2n Äquivalent an Lithiumhydroxid zugegeben. Die Suspension wurde 2 Stunden bei Raumtemperatur gerührt. Die Suspension wurde 15 Minuten zentrifugiert, die flüssige Phase abdekantiert und das Lösungsmittel abgezogen. Das Produkt wurde im Vakuum bei 60°C für 6 Stunden getrocknet. Lithium-2-methoxy-1,1,2,2-tetrafluor-ethansulfonat wurde in Form weißer Kristalle erhalten.

### Beispiel 3

### Bestimmung der Fluorwasserstoff (HF)-Entwicklung in Lithium-2-methoxy-1,1,2,2-tetrafluor-ethansulfonat-basierten Elektrolyten im Vergleich zu LiPF₆

Zur Untersuchung der HF-Entwicklung in Lithium-2-methoxy-1,1,2,2-tetrafluor-ethansulfonat-basierten Elektrolyten wurde das gemäß Beispiel 1 hergestellte Lithium-2-methoxy-1,1,2,2-tetrafluor-ethansulfonat für 24 Stunden mit Hilfe einer Turbomolekularpumpe (Pfeiffer Vacuum) getrocknet. Dabei wurde die Temperatur alle 6 Stunden um 20°C von 60°C auf 120°C erhöht. Anschließend wurde Lithium-2-methoxy-1,1,2,2-tetrafluor-ethansulfonat in einem Gemisch von Ethylencarbonat und Diethylcarbonat (EC:DEC) (beides Ferro Corporation, Batteriegrad/battery grade) im Verhältnis 3:7 bezogen auf die Gewichtsanteile gelöst, so dass sich eine Konzentration von 1 M des Lithiumsalzes ergab.

In einem mit Argon gefüllten Handschuhkasten (MBraun) wurden ca. 400 µL der 1 M Lithium-2-methoxy-1,1,2,2-tetrafluor-ethansulfonat-Lösung in EC:DEC in ein NMR-Rohr aus Glas gegeben und das NMR-Rohr unter Luftausschluss durch Abschmelzen mit einer Mini-Lötlampe versiegelt. Die Probe wurde für 2 Wochen bei 95°C gelagert (Klimaschrank, Binder MK53).

Parallel dazu wurden NMR-Rohre mit 1 molaren Lösungen von Lithium-2-methoxy-1,1,2,2-tetrafluor-ethansulfonat in einem Gemisch von Ethylencarbonat und Adiponitril (Sigma-Aldrich, 99,9%) (EC:ADN) im Verhältnis 1:1 bezogen auf die Gewichtsanteile sowie LiPF₆ (Sigma-Aldrich, Batteriegrad) in einem Gemisch von Ethylencarbonat und Diethylcarbonat im Verhältnis 3:7 bezogen auf die Gewichtsanteile (EC:DEC, 3:7) unter identischen Bedingungen befüllt und ebenfalls bei 95°C für 2 Wochen gelagert.

Anschließend wurden NMR-Spektren von Protonen und Fluor mit Hilfe eines NMR AVANCE III-Spektrometers (200 MHz) von Bruker mit einem Breitband-Probenkopf (5mm) aufgenommen. Hierbei wurde für den Elektrolyten 1 M Lithium-2-methoxy-1,1,2,2-tetrafluor-ethansulfonat in EC:DEC weder im Protonen- noch im Fluorspektrum ein HF-Signal detektiert. Auch die Untersuchung von thermischen Zersetzungsprodukten von 1 M Lithium-2-methoxy-1,1,2,2-tetrafluor-ethansulfonat in EC:ADN zeigte keine Entwicklung von HF, wohingegen für den LiPF₆-basierten Elektrolyten HF sowohl im Protonen- als auch im Fluorspektrum als Zersetzungsprodukt nachgewiesen wurde.

### Beispiel 4

### Analyse der thermischen Stabilität im Vergleich zu LiPF₆

Die Zerfallsprodukte einer 1 M-Lösung von Lithium-2-methoxy-1,1,2,2-tetrafluor-ethansulfonat hergestellt gemäß Beispiel 1 in einem Gemisch von 50 Gew.-% Ethylencarbonat und 50 Gew.-% Diethylcarbonat (EC:DEC, 1:1) wurden gegenüber den Zerfallsprodukten einer 1 M-Lösung von LiPF₆ in einem Gemisch von 30 Gew.-% Ethylencarbonat und 70 Gew.-% Diethylcarbonat (EC:DEC, 3:7) mittels GC-MS bestimmt.

Eine 1 molare Lösung aus Lithium-2-methoxy-1,1,2,2-tetrafluor-ethansulfonat in EC:DEC (1:1) wurde innerhalb eines Handschuhkasten in ein Polyetheretherketon (PEEK)-Gefäß mit Septum gefüllt. Zum Vergleich wurde auch eine 1 molare Lösung aus LiPF₆ (Sigma-Aldrich, Batteriegrad) in EC:DEC (3:7) in ein PEEK Gefäß gefüllt. Anschließend wurden die Gefäße luftdicht verschlossen und bei 95°C für 2 Wochen gelagert. Die Bestimmung der Zersetzungsprodukte erfolgte an einem Clarus 600 Gaschromatographen (Perkin Elmer), angeschlossen an ein Clarus 600 Massenspektrometer (Perkin Elmer).

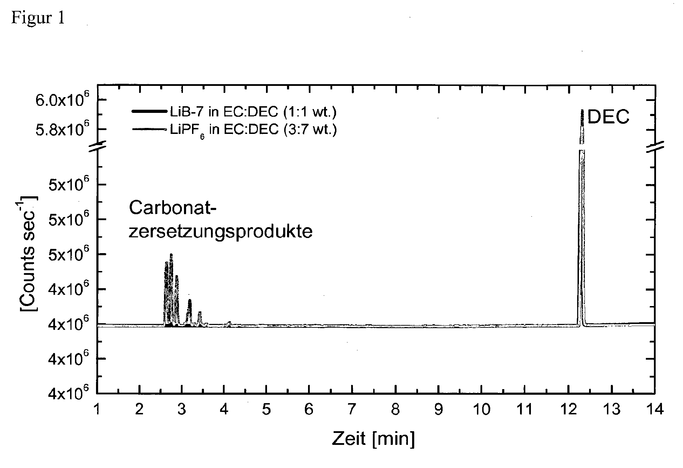

Die Figur 1 zeigt die Zerfallsprodukte der thermischen Alterung bei 95°C. Wie Figur 1 zeigt, waren Zersetzungsprodukte im Falle des LiPF₆-Elektrolyten festzustellen. So wurden für den Elektrolyten 1 M LiPF₆ in EC:DEC anhand der Signale zwischen 2,5 min und 3,5 min Carbonat-Zersetzungsprodukte detektiert. Der Lithium-2-methoxy-1,1,2,2-tetrafluor-ethansulfonat Elektrolyt zeigt dagegen keine Carbonatzersetzungsprodukte nach zweiwöchiger Lagerung bei 95°C. Das Signal nach 12 min ist Diethylcarbonat zuzuordnen.

Dieses Ergebnis bestätigt die thermische Stabilität des Elektrolyten enthaltend Lithium-2-methoxy-1,1,2,2-tetrafluor-ethansulfonat.

### Beispiel 5

Bestimmung der Leitfähigkeit von Lithium-2-methoxy-1,1,2,2-tetrafluor-ethansulfonat

Die Leitfähigkeit von Lithium-2-methoxy-1,1,2,2-tetrafluor-ethansulfonat wurde in unterschiedlichen Lösungsmitteln in einem Temperaturbereich von - 40°C bis + 60°C bestimmt.

Das gemäß Beispiel 1 hergestellte Lithium-2-methoxy-1,1,2,2-tetrafluor-ethansulfonat wurde für 24 Stunden mit Hilfe einer Turbomolekularpumpe (Pfeiffer Vacuum) getrocknet. Dabei wurde die Temperatur alle 6 Stunden um 20°C von 60°C auf 120°C erhöht. Mischungen von 50 Gew.-% Ethylencarbonat (EC) (Ferro Corporation, Batteriegrad) und jeweils 50 Gew.-% Diethylcarbonat (DEC) (Ferro Corporation, Batteriegrad), Dimethylcarbonat (DMC) (Ferro Corporation, Batteriegrad) oder Adiponitril (ADN) (Sigma-Aldrich, 99,9%) wurden vorgelegt. In diesen Lösungsmittelgemischen wurden die jeweils benötigte Menge Lithium-2-methoxy-1,1,2,2-tetrafluor-ethansulfonat gelöst, so dass sich eine Konzentration von 1 M des Lithiumsalzes ergab.

Die Leitfähigkeit der Elektrolyte wurde unter Verwendung von Platin-Leitfähigkeitsmesszellen (Amel Glassware, Zellkonstante 1 cm⁻¹) mit einem Potentiostaten (Solartron 1280A) in Verbindung mit einer Impedanzmesseinheit (Soartron 1260) in einem Temperaturbereich von - 40°C bis + 60°C (Klimaschrank, Binder MK53) untersucht. Hierzu wurden die Leitfähigkeitsmesszellen zunächst auf 60°C erwärmt und dann in Temperaturintervallen von 5°C auf -40°C gekühlt.

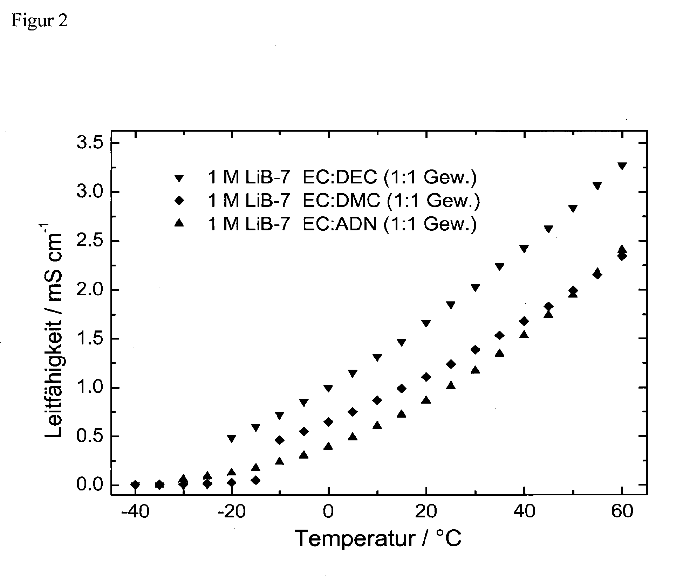

Wie in Figur 2 gezeigt ist, wurde die höchste Leitfähigkeit im Temperaturbereich von -20°C - +60°C in dem Lösungsmittelgemisch EC:DEC 1:1 erreicht. Die Leitfähigkeiten für die verschiedenen Lösungsmittelgemische bei 20°C betrugen für ein 1:1-Gemisch Ethylencarbonat und Diethylcarbonat 1,67 mS cm⁻¹, für ein 1:1-Gemisch Ethylencarbonat und Dimethylcarbonat (EC:DMC 1:1) 1,1 mS cm⁻¹ und für ein 1:1-Gemisch Ethylencarbonat und Adiponitril (EC:ADN 1:1) 0,86 mS cm⁻¹.

Dies zeigt, dass Lithium-2-methoxy-1,1,2,2-tetrafluor-ethansulfonat in den üblichen Carbonat-Lösungsmitteln eine ausreichende Leitfähigkeit bei 20°C aufweist.

### Beispiel 6

### Bestimmung der elektrochemischen Stabilität von Lithium-2-methoxy-1,1,2,2-tetrafluor-ethansulfonat

Die elektrochemische Stabilität einer 1 M-Lösung von Lithium-2-methoxy-1,1,2,2-tetrafluor-ethansulfonat in einem Gemisch von Ethylencarbonat und Diethylcarbonat im Verhältnis 1:1 bezogen auf die Gewichtsanteile (EC:DEC, 1:1) gegenüber der Stabilität einer 1 M-Lösung von LiPF₆ (Sigma-Aldrich, Batteriegrad) in einem Gemisch von Ethylencarbonat und Diethylcarbonat im Verhältnis 3:7 bezogen auf die Gewichtsanteile (EC:DEC, 3:7) wurde mittels der sogenannten Linear Sweep-Voltammetrie (LSV) bestimmt. Bei diesem Verfahren erfolgt eine kontinuierliche Änderung der Elektrodenspannung (linear sweep). Hierzu wurde das gemäß Beispiel 1 hergestellte Lithium-2-methoxy-1,1,2,2-tetrafluor-ethansulfonat für 24 h mit Hilfe einer Turbomolekularpumpe (Pfeiffer Vacuum) getrocknet, wobei die Temperatur alle 6 Stunden um 20°C von 60°C auf 120°C erhöht wurde.

Die Versuche wurden in einer 3-Elektrodenanordnung in modifizierte Swagelok®-T-Stücke (Rohrverbinder, Edelstahlkörper) mit einer Platinelektrode (eDAQ, Modell ET075, 1 mm Durchmesser) als Arbeitselektrode und Lithiumfolie (12 mm bzw. 7 mm Durchmesser, Chemetall) als Gegen- und Referenzelektrode durchgeführt. Dazu wurde der Zellkörper mit einer einseitig silikonierten Polyester-Folie (Mylar®, PPI-SP 914, 100 µm) ausgekleidet und die Elektroden in den Zellkörper eingeführt. Die Elektroden wurden mit einem Vlies (Freudenberg®, FS2226E, 6 Lagen) separiert, welches mit dem entsprechenden Elektrolyten getränkt war. Die Vorschubgeschwindigkeit betrug 1 mV s⁻¹.

Als kathodisches Stabilitätslimit, das Potential bei welchem eine Reduktion einsetzt, wurde jenes Potential definiert, bei welchem die Stromdichte - 0,1 mA cm⁻² unterschritt und als anodisches Stabilitätslimit, das Potential bei welchem Oxidation einsetzt, jenes Potential, bei welchem die Stromdichte + 0,1 mA cm⁻² überschritt. Insbesondere die anodische Stabilität hängt entscheidend von der Stabilität des verwendeten Elektrolyten ab.

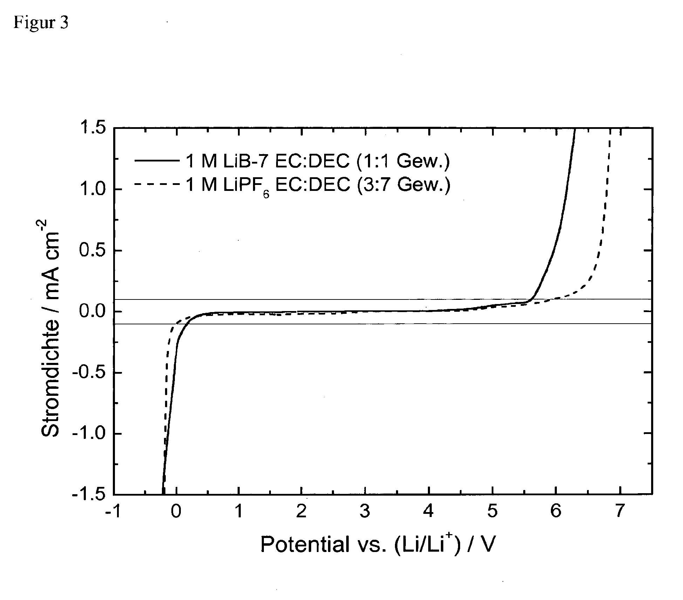

Wie in Figur 3 gezeigt ist, wurde bei dem Elektrolyt Lithium-2-methoxy-1,1,2,2-tetrafluor-ethansulfonat in EC:DEC 1:1 das kathodische Stabilitätslimit bei 0,2 V erreicht, das von LiPF₆ in EC:DEC 3:7 bei 0,0 V. Die anodische Stabilität von Lithium-2-methoxy-1,1,2,2-tetrafluor-ethansulfonat in EC:DEC 1:1 war mit 5,6 V nur geringfügig um ca. 0,3 V geringer als jene von 5,9 V von LiPF₆ in EC:DEC 3:7. Diese anodische Stabilität ist völlig ausreichend für die Verwendung des Elektrolyten in Kombination mit Hochvoltkathodenmaterialien.

Dieses Ergebnis zeigt, dass Lithium-2-methoxy-1,1,2,2-tetrafluor-ethansulfonat in den üblichen Carbonat-Lösungsmitteln eine für sämtlichen elektrochemischen Anwendungen ausreichend gute elektrochemischen Stabilität aufweist.

### Beispiel 7

### Bestimmung des Korrosionsverhaltens von Lithium-2-methoxy-1,1,2,2-tetrafluor-ethansulfonat

Das Korrosionsverhalten von Lithium-2-methoxy-1,1,2,2-tetrafluor-ethansulfonat in Bezug auf Aluminium wurde im Vergleich zu dem als korrosiv bekannten Lithium Bis(Trifluoromethansulfonyl)imid (LiTFSI) bestimmt. Aluminium wird an der Kathodenseite als Stromsammler eingesetzt und ist in dem Potentialbereich, in dem Lithium-Ionen Batterien betrieben werden, thermodynamisch nicht stabil. Daher ist es wichtig, dass der Elektrolyt in der Lage ist, eine Schutzschicht auf Aluminium auszubilden, welche die Korrosion des Stromsammlers verhindert. Bei Elektrolyten, die nicht in der Lage sind eine Schutzschicht auf Aluminium aufzubauen, wie LiTFSI, steigt die Stromdichte aufgrund von Aluminiumkorrosion während des potentiostatischen Schrittes an, wohingegen sie abnimmt, wenn keine Aluminiumkorrosion stattfindet.

Verwendet wurden 1 M-Lösungen von Lithium-2-methoxy-1,1,2,2-tetrafluor-ethansulfonat, LiPF₆ und LiTFSI in einem Gemisch von Ethylencarbonat und Diethylcarbonat im Verhältnis 3:7 bezogen auf die Gewichtsanteile (EC:DEC, 3:7). Das gemäß Beispiel 1 hergestellte Lithium-2-methoxy-1,1,2,2-tetrafluor-ethansulfonat wurde zuvor für 24 Stunden mit Hilfe einer Turbomolekularpumpe (Pfeiffer Vacuum) getrocknet, wobei die Temperatur alle 6 Stunden um 20°C von 60°C auf 120°C erhöht wurde.

Zur Messung der Aluminiumkorrosionseigenschaften der Elektrolyten wurden 3-Elektrodenzellen (modifizierte Swagelok®-T-Stücke, Edelstahlkörper) mit Aluminiumfolie (12 mm bzw. 7 mm Durchmesser) als Arbeitselektrode und Lithiumfolie (12 mm bzw. 7 mm Durchmesser, Chemetall) als Gegen- und Referenzelektrode hergestellt. durchgeführt. Dazu wurde der Zellkörper mit einer einseitig silikonierten Polyester-Folie (Mylar®, PPI-SP 914, 100 µm) ausgekleidet und die Elektroden in den Zellkörper eingeführt. Die Elektroden wurden mit einem Vlies (Freudenberg®, FS2226E, 6 Lagen) separiert, welches mit dem entsprechenden Elektrolyten getränkt war.

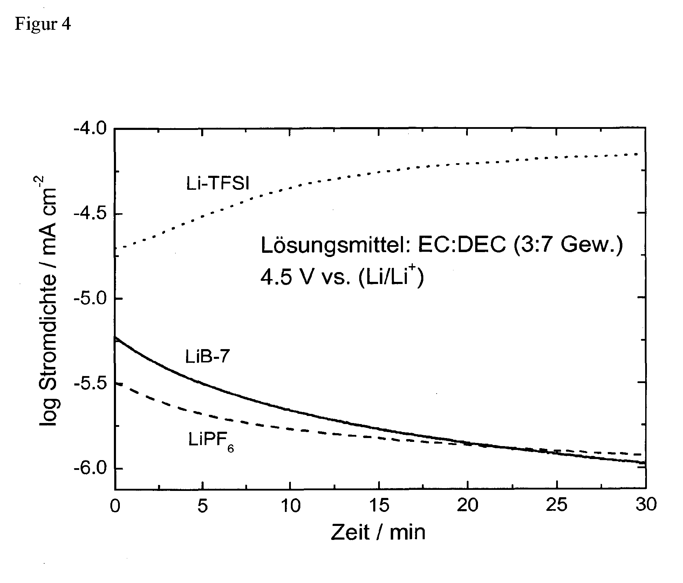

Bei der Leerlaufspannung der Zelle startend wurde das Potential in 100 mV Schritten mit 1 mV s⁻¹ erhöht und anschließend das jeweilige Potential für eine Stunde gehalten. Der Verlauf der Stromdichte während dieses sogenannten potentiostatischen Schrittes bei 4,5 V ist in Figur 4 dargestellt.

Wie in Figur 4 gezeigt ist, stieg die Stromdichte bei einem Potential von 4,5 V vs. Li/Li⁺ bei der Verwendung von LiTFSI als Leitsalz an. Dieser Effekt ist auf Aluminiumkorrosion zurückzuführen. Demgegenüber nahm die Stromdichte bei der Messung der auf Lithium-2-methoxy-1,1,2,2-tetrafluor-ethansulfonat und LiPF₆ basierenden Elektrolyten ab.

Dieses Ergebnis zeigt, dass der Lithium-2-methoxy-1,1,2,2-tetrafluor-ethansulfonat-Elektrolyt wie der LiPF₆-Elektrolyt in der Lage ist, eine Korrosion von Aluminium durch die Ausbildung einer Schutzschicht bei einem Potential von 4,5 V zu vermeiden.

### Beispiel 8

### Bestimmung des Zyklisierverhaltens von Lithium-2-methoxy-1,1,2,2-tetrafluor-ethansulfonat

Das Zyklisierverhalten einer 1 M-Lösung von Lithium-2-methoxy-1,1,2,2-tetrafluor-ethansulfonat in einem Gemisch von Ethylencarbonat und Diethylcarbonat im Verhältnis 1:1 bezogen auf die Gewichtsanteile wurde im Vergleich zum Standardelektrolyten einer 1 M-Lösung von LiPF₆ in Gemisch von Ethylencarbonat und Diethylcarbonat im Verhältnis 3:7 bezogen auf die Gewichtsanteile bestimmt. Das gemäß Beispiel 1 hergestellte Lithium-2-methoxy-1,1,2,2-tetrafluor-ethansulfonat wurde zuvor für 24 Stunden mit Hilfe einer Turbomolekularpumpe (Pfeiffer Vacuum) getrocknet, wobei die Temperatur alle 6 Stunden um 20°C von 60°C auf 120°C erhöht wurde.

Hierzu wurden 3-Elektrodenzellen (modifizierte Swagelok®-T-Stücke, Edelstahlkörper) mit Timrex T44 Graphit (TIMCAL Graphite&Carbon, 12 mm Durchmesser) als Anode, eine ∼8-fach kapazitativ überdimensionierten NCA-Kathode (Lithium-Nickel-Cobalt-Aluminium-Oxid, Li(Ni_{0,8}Co_{0,15}Al_{0,05})O₂, 12 mm Durchmesser) als Lithiumquelle und Lithiummetall (7 mm Durchmesser, Chemetall) als Referenzelektrode hergestellt. Dazu wurde der Zellkörper mit einer einseitig silikonierten Polyester-Folie (Mylar®, PPI-SP 914, 100 µm) ausgekleidet und die Elektroden in den Zellkörper eingeführt. Die Elektroden wurden mit einem Vlies (Freudenberg®, FS2226E, 6 Lagen) separiert, welches mit dem entsprechenden Elektrolyten getränkt war. Die Zellen wurden zuerst für 3 Zyklen mit einer Rate von C/5 formiert und danach für 20 Zyklen in einem Potentialbereich von 0,025 V - 1,5 V bei 1C ge- und entladen.

Beim Laden wurde jeweils nach Erreichen der Ladeschlussspannung noch für eine Stunde mit Konstantspannung (25 mV) weitergeladen. Anschließend wurde ein sogenannter C-Raten-Test durchgeführt, bei welchem jeweils mit einer C-Rate von C/2 und anschließend eine Stunde Konstantspannung (25 mV) geladen und bei unterschiedlichen C-Raten, von C/5 bis 5C, entladen wurde. Nach dem C-Raten-Test wurden die Zellen für 5 Zyklen mit C/5 und anschließend für 30 Zyklen mit 1C ge- und entladen. Beim Laden wurde wie bereits zuvor jeweils ein Konstantspannungsschritt von einer Stunde durchgeführt.

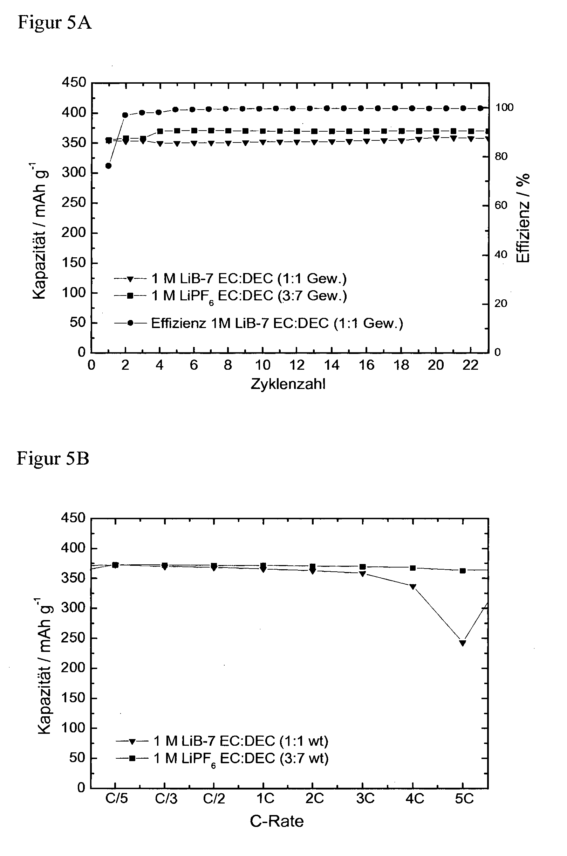

Wie die Figur 5A zeigt, wies die Zelle mit dem Elektrolyt 1 M Lithium-2-methoxy-1,1,2,2-tetrafluor-ethansulfonat in EC:DEC 1:1 eine Anfangskapazität von ca. 350 mAh g⁻¹ nach dem Formieren der Zelle auf, die kontinuierlich mit der Anzahl der Zyklen auf ca. 360 mAh g⁻¹ im 20. Zyklus stieg. Dies weist daraufhin, dass es zu einer Verbesserung der Benetzung der Elektroden mit steigender Zyklenzahl kommt. Die Zellen mit dem Elektrolyten LiPF₆ erreichten nach den 3. Formierungszyklen die theoretische Kapazität von ca. 370 mAh g⁻¹.

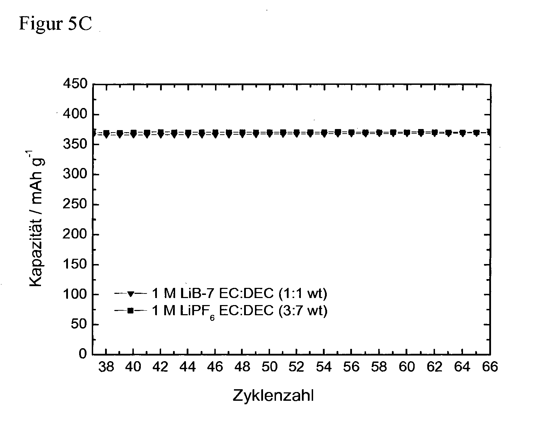

Wie in Figur 5B gezeigt ist, zeigten beim C-Raten-Test beide Zellen bis zu einer C-Rate von 2C vergleichbare Kapazitäten mit einem Unterschied von weniger als 10 mAh g⁻¹. Erst ab einer hohen C-Rate von > 3C kam es zu signifikanten Kapazitätsunterschieden. Dies ist auf die geringere Leitfähigkeit von 1 M Lithium-2-methoxy-1,1,2,2-tetrafluor-ethansulfonat in EC:DEC 1:1 von 1,7 mS cm⁻¹ (20°C) im Vergleich zu ca. 6,5 mS cm⁻¹ (20°C) für 1 M LiPF₆ in EC:DEC 3:7 zurückzuführen. Wie in Figur 5C gezeigt ist, erreichte nach dem C-Raten-Test und 5 Zyklen bei C/5 auch die Zelle mit 1 M Lithium-2-methoxy-1,1,2,2-tetrafluor-ethansulfonat in EC:DEC 1:1 die theoretische Kapazität und wies eine hervorragende Zyklenstabilität über 30 Zyklen auf.

Die Ergebnisse zeigen, dass Elektrolyte mit Lithium-2-methoxy-1,1,2,2-tetrafluor-ethansulfonat als Leitsalz zwar eine etwas geringere Leitfähigkeit und Oxidationsstabilität im Vergleich zu Elektrolyten mit dem Standardsalz LiPF₆ aufwiesen, jedoch eine hervorragende Zyklenstabilität und keine HF-Entwicklung nach thermischer Alterung bei 95°C.

Diese Ergebnisse zeigen, dass Lithium-2-methoxy-1,1,2,2-tetrafluor-ethansulfonat eine Möglichkeit bietet, LiPF₆ als Leitsalz in Lithium-Ionen Batterien zu ersetzen.

## Patentansprüche

1. Lithium-2-methoxy-1,1,2,2-tetrafluor-ethansulfonat.

2. Verwendung von Lithium-2-methoxy-1,1,2,2-tetrafluor-ethansulfonat als Leitsalz in Elektrolyten für Lithium-basierte Energiespeicher, vorzugsweise ausgewählt aus der Gruppe umfassend Lithium-Batterien, Lithium-Ionen-Batterien, Lithium-Ionen-Akkumulatoren, Lithium-Polymer-Batterien und/oder Lithium-Ionen-Kondensatoren.

3. Elektrolyt für einen Lithium-basierten Energiespeicher umfassend Lithium-2-methoxy-1,1,2,2-tetrafluor-ethansulfonat.

4. Elektrolyt nach Anspruch 3, **dadurch gekennzeichnet, dass** der Elektrolyt ein aprotisches Lösungsmittel, eine ionische Flüssigkeit und/oder eine Polymermatrix umfasst.

5. Elektrolyt nach Anspruch 3 oder 4, **dadurch gekennzeichnet, dass** das aprotische Lösungsmittel ausgewählt ist aus der Gruppe umfassend Ethylencarbonat, Propylencarbonat, Diethylcarbonat, Dimethylcarbonat, Ethylmethylcarbonat, Acetonitril, Glutaronitril, Adiponitril, Pimelonitril, gamma-Butyrolacton, gamma-Valerolacton, Dimethoxyethan, 1,3-Dioxalan, Methylacetat und/oder Mischung davon, vorzugsweise aus der Gruppe umfassend Ethylencarbonat, Diethylcarbonat, Dimethylcarbonat und/oder deren Mischungen.

6. Elektrolyt nach einem der vorherigen Ansprüche, **dadurch gekennzeichnet, dass** das aprotische Lösungsmittel eine Mischung aus Ethylencarbonat und wenigstens einen weiteren aprotischen Lösungsmittel vorzugsweise Diethylcarbonat umfasst, bevorzugt im Verhältnis im Bereich von ≥ 1:9 bis ≤ 9:1, bevorzugt im Bereich von ≥ 3:7 bis ≤ 7:3, vorzugsweise im Bereich von ≥ 3:7 bis ≤ 1:1.

7. Elektrolyt nach einem der vorherigen Ansprüche, **dadurch gekennzeichnet, dass** die Konzentration von Lithium-2-methoxy-1,1,2,2-tetrafluor-ethansulfonat im Elektrolyten im Bereich von ≥ 0,5 M bis ≤ 2,5 M, vorzugsweise im Bereich von ≥ 0,65 M bis ≤ 2 M, besonders bevorzugt im Bereich von ≥ 1 M bis ≤ 1,5 M, liegt.

8. Lithium-basierter Energiespeicher, vorzugsweise Lithium-Batterie, Lithium-Ionen-Batterie, Lithium-Ionen-Akkumulator, Lithium-Polymer-Batterie oder Lithium-Ionen-Kondensator, umfassend Lithium-2-methoxy-1,1,2,2-tetrafluor-ethansulfonat.

9. Verfahren zur Herstellung von Lithium-2-methoxy-1,1,2,2-tetrafluor-ethansulfonat, umfassend die folgenden Schritte:
a) Umsetzen von Difluorfluorsulfonylacetylfluorid mit Triethylamintrihydrofluorid oder Tetramethylammoniumfluorid zu Triethylammonium-2-sulfonylfluorid-tetrafluorethanolat oder Tetramethylammonium-2-sulfonylfluorid-tetrafluorethanolat,
b) Methylierung von Triethylammonium-2-sulfonylfluorid-tetrafluorethanolat oder Tetramethylammonium-2-sulfonylfluorid-tetrafluorethanolat zu 2-Methoxy-1,1,2,2-tetrafluor-ethansulfonylfluorid, und
c) Umsetzen von 2-Methoxy-1,1,2,2-tetrafluor-ethansulfonylfluorid mit Lithiumhydroxid zu Lithium-2-methoxy-1,1,2,2-tetrafluorethansulfonat.

10. Ionische Flüssigkeit umfassend 2-Methoxy-1,1,2,2-tetrafluor-ethansulfonat als Anion und ein organisches Kation ausgewählt aus der Gruppe umfassend Alkylammonium-, Pyridinium-, Pyrazolium-, Pyrrolium-, Pyrrolinium-, Piperidinium-, Pyrrolidinium-, Imidazolium- und/oder Sulfoniumverbindungen, wobei das Kation vorzugsweise ausgewählt ist aus der Gruppe umfassend N-butyl-N-methylpyrrolidinium, N-methyl-N-propylpyrrolidinium, 1-Ethyl-3-methylimidazolium, 1-Ethyl-2,3-dimethylimidazolium und/oder 1-Butyl-3-methylimidazolium.

## Claims

1. Lithium 2-methoxy-1,1,2,2-tetrafluoroethane-sulphonate.

2. Use of lithium 2-methoxy-1,1,2,2-tetrafluoro-ethanesulphonate as a conductive salt in electrolytes for lithium-based energy storage device, preferably selected from the group comprising lithium batteries, lithium-ion batteries, lithium-ion accumulators, lithium polymer batteries and/or lithium-ion capacitors.

3. Electrolyte for a lithium-based energy storage device comprising lithium 2-methoxy-1,1,2,2-tetrafluoroethanesulphonate.

4. Electrolyte according to claim 3, **characterized in that** the electrolyte comprises an aprotic solvent, an ionic liquid and/or a polymer matrix.

5. Electrolyte according to claim 3 or 4, **characterized in that** the aprotic solvent is selected from the group comprising ethylene carbonate, propylene carbonate, diethyl carbonate, dimethyl carbonate, ethyl methyl carbonate, acetonitrile, glutaronitrile, adiponitrile, pimelonitrile, gamma-butyrolactone, gamma-valerolactone, dimethoxyethane, 1,3-dioxolane, methyl acetate and/or a mixture thereof, preferably from the group comprising ethylene carbonate, diethyl carbonate, dimethyl carbonate and/or mixtures thereof.

6. Electrolyte according to any of the preceding claims, **characterized in that** the aprotic solvent comprises a mixture of ethylene carbonate and at least one further aprotic solvent, preferably diethyl carbonate, preferably in a ratio in the range from ≥ 1:9 to ≤ 9:1, preferably in the range from ≥ 3:7 to ≤ 7:3, preferably in the range from ≥ 3:7 to ≤ 1:1.

7. Electrolyte according to any of the preceding claims, **characterized in that** the concentration of lithium 2-methoxy-1,1,2,2-tetrafluoroethanesulphonate in the electrolyte is in the range from ≥ 0.5 M to ≤ 2.5 M, preferably in the range from ≥ 0.65 M to ≤ 2 M, more preferably in the range from ≥ 1 M to ≤ 1.5 M.

8. Lithium-based energy storage device, preferably lithium battery, lithium-ion battery, lithium-ion accumulator, lithium polymer battery or lithium-ion capacitor, comprising lithium 2-methoxy-1,1,2,2-tetrafluoroethanesulphonate.

9. Process for preparing lithium 2-methoxy-1,1,2,2-tetrafluoroethanesulphonate, comprising the following steps:
a) reacting difluorofluorosulphonylacetyl fluoride with triethylamine trihydrofluoride or tetramethylammonium fluoride to give triethyl-ammonio-2-sulphonyl fluoride tetrafluoroethoxide or tetramethylammonio-2-sulphonyl fluoride tetrafluoroethoxide,
b) methylating triethylammonio-2-sulphonyl fluoride tetrafluoroethoxide or tetramethylammonio-2-sulphonyl fluoride tetrafluoroethoxide to give 2-methoxy-1,1,2,2-tetrafluoroethanesulphonyl fluoride, and
c) reacting 2-methoxy-1,1,2,2-tetrafluoroethane-sulphonyl fluoride with lithium hydroxide to give lithium 2-methoxy-1,1,2,2-tetrafluoro-ethanesulphonate.

10. Ionic liquid comprising 2-methoxy-1,1,2,2-tetra-fluoroethanesulphonate as anion and an organic cation selected from the group comprising alkylammonium, pyridinium, pyrazolium, pyrrolium, pyrrolinium, piperidinium pyrrolidinium, imidazolium and/or sulphonium compounds, the cation preferably being selected from the group comprising N-butyl-N-methylpyrrolidinium, N-methyl-N-propylpyrrolidinium, 1-ethyl-3-methylimidazolium, 1-ethyl-2,3-dimethylimidazolium and/or 1-butyl-3-methylimidazolium.

## Revendications

1. 2-Méthoxy-1,1,2,2-tétrafluoro-éthanesulfonate de lithium.

2. Utilisation du 2-méthoxy-1,1,2,2-tétrafluoro-éthanesulfonate de lithium comme sel conducteur dans des électrolytes pour des accumulateurs d'énergie à base de lithium, de préférence choisis dans le groupe comprenant des batteries lithium, des batteries lithium-ion, des accumulateurs lithium-ion, des batteries lithium-polymère et/ou des condensateurs lithium-ion.

3. Électrolyte pour un accumulateur d'énergie à bas de lithium, comprenant du 2-méthoxy-1,1,2,2-tétrafluoro-éthanesulfonate de lithium.

4. Électrolyte selon la revendication 3, **caractérisé en ce que** l'électrolyte comprend un solvant aprotique, un liquide ionique et/ou une matrice polymère.

5. Électrolyte selon la revendication 3 ou 4, **caractérisé en ce que** le solvant aprotique est choisi dans le groupe comprenant le carbonate d'éthylène, le carbonate de propylène, le carbonate de diéthyle, le carbonate de diméthyle, le carbonate d'éthyle et de méthyle, l'acétonitrile, le glutaronitrile, l'adiponitrile, le pimélonitrile, la gamma-butyrolactone, la gamma-valérolactone, le diméthoxyéthane, le 1,3-dioxalane, l'acétate de méthyle et/ou un mélange de ceux-ci, de préférence dans le groupe comprenant le carbonate d'éthylène, le carbonate de diéthyle, le carbonate de diméthyle et/ou des mélanges de ceux-ci.

6. Électrolyte selon l'une quelconque des revendications précédentes, **caractérisé en ce que** le solvant aprotique comprend un mélange de carbonate d'éthylène et d'au moins un autre solvant aprotique, de préférence le carbonate de diéthyle, de préférence en un rapport dans la plage de ≥ 1:9 à ≤ 9:1, de préférence dans la plage de ≥ 3:7 à ≤ 7:3, encore mieux dans la plage de ≥ 3:7 à ≤ 1:1.

7. Électrolyte selon l'une quelconque des revendications précédentes, **caractérisé en ce que** la concentration de 2-méthoxy-1,1,2,2-tétrafluoro-éthanesulfonate de lithium dans l'électrolyte se situe dans la plage de ≥ 0,5 M à ≤ 2,5 M, de préférence dans la plage de ≥ 0,65 M à ≤ 2 M, de façon particulièrement préférée dans la plage de ≥ 1 M à ≤ 1,5 M.

8. Accumulateur d'énergie à base de lithium, de préférence batterie lithium, batterie lithium-ion, accumulateur lithium-ion, batterie lithium-polymère ou condensateur lithium-ion, comprenant du 2-méthoxy-1,1,2,2-tétrafluoro-éthanesulfonate de lithium.

9. Procédé pour la préparation de 2-méthoxy-1,1,2,2-tétrafluoro-éthanesulfonate de lithium, comprenant les étapes suivantes :
a) mise en réaction de fluorure de difluorofluoro-sulfonylacétyle avec du fluorhydrate de triéthyl-amine ou du fluorure de tétraméthylammonium pour l'obtention de 2-sulfonylfluorure-tétrafluoro-éthanolate de triéthylammonium ou de 2-sulfonyl-fluorure-tétrafluoroéthanolate de tétraméthyl-ammonium,
b) méthylation de 2-sulfonylfluorure-tétrafluoro-éthanolate de triéthylammonium ou de 2-sulfonyl-fluorure-tétrafluoroéthanolate de tétraméthyl-ammonium pour l'obtention de fluorure de 2-méthoxy-1,1,2,2-tétrafluoro-éthanesulfonyle, et
c) mise en réaction de fluorure de 2-méthoxy-1,1,2,2-tétrafluoro-éthanesulfonyle avec de l'hydroxyde de lithium pour l'obtention de 2-méthoxy-1,1,2,2-tétrafluoroéthanesulfonate de lithium.

10. Liquide ionique comprenant du 2-méthoxy-1,1,2,2-tétrafluoro-éthanesulfonate comme anion et un cation organique choisi dans le groupe comprenant des composés alkylammonium, pyridinium, pyrazolium, pyrrolium, pyrrolinium, pipéridinium, pyrrolidinium, imidazolium et/ou sulfonium, le cation étant de préférence choisi dans le groupe comprenant le N-butyl-N-méthylpyrrolidinium, le N-méthyl-N-propylpyrrolidinium, le 1-éthyl-3-méthylimidazolium, le 1-éthyl-2,3-diméthylimidazolium et/ou le 1-butyl-3-méthylimidazolium.
